# EUROPEAN PATENT SPECIFICATION

(11) **EP 1 897 605 B1**
(45) Date of publication and mention of the grant of the patent: **09.04.2014**
(21) Application number: 07115700.2
(22) Date of filing: 05.09.2007
(51) Int. Cl.: B01D 65/10, A61M 1/16

(54) **Method and device for testing the integrity of dialysis circuit filters**
Verfahren und Vorrichtung zum Testen der Unversehrtheit von Dialysekreislauffiltern
Procédé et dispositif pour tester l'intégrité de filtres de circuit de dialyse

(30) Priority: 05.09.2006 IT BO20060625
(43) Date of publication of application: 12.03.2008
(73) Proprietor: Bellco S.r.l., Mirandola (IT)
(72) Inventor: Albertini Milco, 41037, Mirandola (IT); Cavani, Silvia, 41100, Modena (IT); Cianciavicchia, Domenico, 64040, Cavuccio (IT); Fiorenzi, Andrea, 41013, Castelfranco Emilia (IT); Pradelli, Alessandro, 41034, Finale Emilia (IT); Vaira, Fabrizio, 71100, Foggia (IT)
(74) Representative: Bergadano, Mirko

(56) References cited:
- EP-A- 0 763 367
- WO-A-2007/003980
- DE-A1- 3 442 744
- DE-A1- 19 700 466

## Description

The present invention relates to a method of testing the integrity of dialysis circuit filters.

Haemodialysis is a blood-purifying method for restoring hydrosaline balance and eliminating surplus water and toxic substances accumulating in the body as a result of kidney failure, by releasing them to an electrolytic fluid similar to that of normal plasma not containing them. Here and hereinafter, this fluid is referred to as "dialysis solution". In dialysis, blood drawn from the patient's arm flows along the so-called artery line into the dialyzer, out of the dialyzer along the so-called vein line, and is restored, purified, to the patient.

In haemodiafiltration, to which the following description refers purely by way of example, blood is purified by both diffusion and convection. Purification by diffusion is based on the presence of a concentration gradient between the two solutions on either side of the membrane, which causes solutes to pass to the lower-concentration side; while purification by convection is based on generating in the dialysis fluid a negative hydraulic pressure with respect to the blood. Because the dialysis membrane is partly permeable by solutes, plasma water flow is accompanied by a stream of plasma solutes compatible in size with the porosity of the membrane.

Part of the plasma flow through the membrane is replaced with a sterile substitution fluid, which is added to the extracorporeal blood flow either upstream (pre-dilution) or downstream (post-dilution) from the dialyzer.

The substitution fluid may be formed "on-line" from the dialysis fluid, and, since the dialysis fluid is not always sterile and devoid of pyrogenous substances, is formed by filtering the dialysis fluid using one or two filters located along the dialysis circuit, upstream from the dialyzer, and comprising two chambers separated by a hydrophilic membrane.

Consequently, the integrity of the filters must be monitored periodically to avoid using substitution fluid of below-standard sterility.

Apparatuses and processes for testing filters are described in Patent Applications EP0763367A, WO2007/003980 A and DE19700466A1.

It is an object of the present invention to provide a straightforward, low-cost method of testing the membrane integrity of substitution fluid filters.

According to the present invention, there is provided a method of testing the integrity of a membrane as claimed in claim 1.

It is a further object of the present invention to provide a unit for testing the integrity of at least one filter in a dialysis solution circuit as claimed in claim 3.

A number of non-limiting embodiments of the invention will be described by way of example with reference to the accompanying drawings, in which:
Figure 1 shows a schematic of part of a dialysis machine for illustrative purpose only;
Figure 2 shows a schematic of part of a dialysis machine for illustrative purpose only;
Figure 3 shows a schematic of part of a dialysis machine in accordance with an embodiment of the present invention.

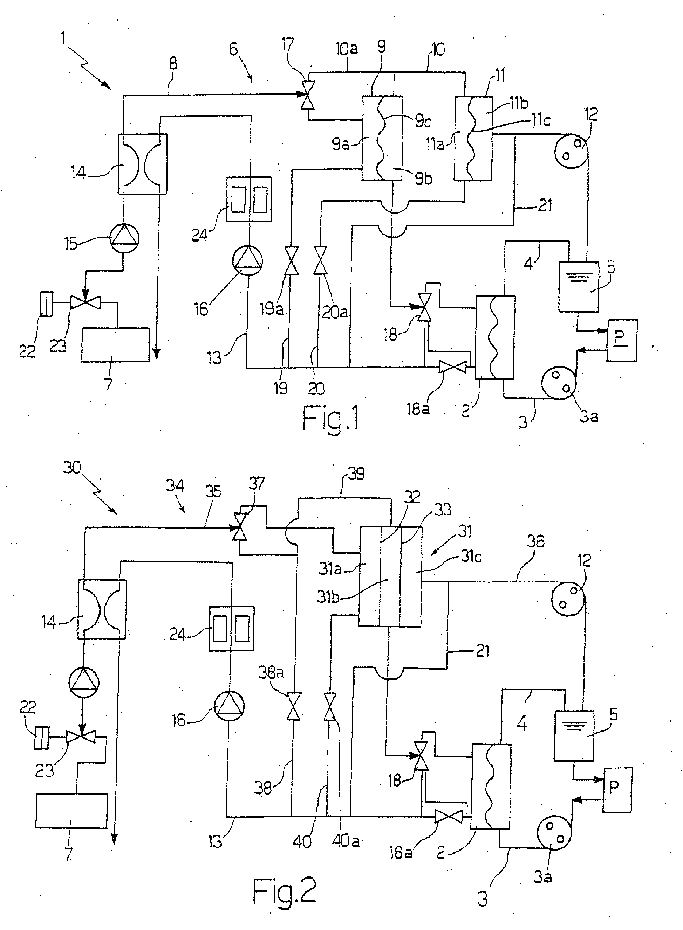

Number 1 in Figure 1 indicates as a whole the dialysis machine (only shown partly).

Machine 1 comprises a known haemodialysis filter 2 (not described in detail); an artery line 3 for feeding blood from a patient P to filter 2; a pump 3a fitted to artery line 3 to ensure blood flow; a vein line 4 for feeding blood from filter 2 to patient P; a drip chamber 5 located along vein line 4; and a dialysis solution circuit 6.

Dialysis solution circuit 6 comprises a preparation device 7; an inflow branch 8 for feeding dialysis solution to filter 2; a first sterile filter 9 along inflow branch 8; a substitution-fluid line 10 for feeding substitution fluid from first sterile filter 9 to drip chamber 5; a second sterile filter 11 along substitution-fluid line 10; a pump 12 located along substitution-fluid line 10, downstream from second sterile filter 11; a dialysis solution outflow branch 13 from filter 2; and a flow gauge 14 through which inflow branch 8 and outflow branch 13 extend. Inflow branch 8 and outflow branch 13 are fitted with respective pumps 15 and 16.

First and second sterile filters 9, 11 each comprise a first chamber 9a, 11a and a second chamber 9b, 11b separated by a hydrophilic membrane 9c, 11c for preventing any bacteria or endotoxins in the dialysis solution from passing from the first to the second chamber of the filter.

Inflow branch 8 comprises a first bypass solenoid valve 17 for bypassing the first chamber of first sterile filter 9 and connecting inflow branch 8, by means of a connecting line 10a, to second chamber 9b of the first sterile filter and, hence, to substitution-fluid line 10, which, in the example shown, extends from second chamber 9b of filter 9. Inflow branch 8 also comprises a second bypass solenoid valve 18 for bypassing haemodialysis filter 2 and connecting inflow branch 8 directly to outflow branch 13, either upstream or downstream from a solenoid valve 18a, depending on the operating mode employed.

Machine 1 comprises a first drain line 19 connecting first chamber 9a of first sterile filter 9 to outflow branch 13; and a second drain line 20 connecting first chamber 11a of second sterile filter 11 to outlet branch 13. Drain lines 19, 20 are fitted with respective solenoid spill valves 19a, 20a, which are opened periodically to wash the membranes of filters 9 and 11 and prevent accumulated bacteria and endotoxins from impairing operation of the filters.

Machine 1 also comprises a test line 21 connecting substitution-fluid line 10, downstream from second sterile filter 11, to outflow branch 13.

Finally, machine 1 comprises an antibacterial filter 22 for filtering outside air; a solenoid valve 23 for switching the fluid source of inflow branch 8 from preparation device 7 to antibacterial filter 22; and an air sensor 24 located along outflow branch 13, downstream from the connections to drain lines 19, 20 and test line 21. Sensor 24 is an ultrasound, continuous-reading type, but may be any of various other types, e.g. optical. The type of sensor 24 is of only marginal importance, and is in no way limiting as regards the present invention.

In actual use, once dialysis treatment is completed, machine 1 is switched from dialysis mode to wash/test mode. After dialysis solution circuit 6 has been flushed with an aqueous solution, e.g. the dialysis solution itself, solenoid valve 23 is switched to antibacterial filter 22 to feed circuit 6 with air from antibacterial filter 22 as opposed to the dialysis solution from preparation device 7. At the same time, the outlet of second chamber 9b of first filter 9 is closed by closing solenoid valve 18a and switching bypass solenoid valve 18 to the circuit portion upstream from solenoid valve 18a, the outlet of first chamber 11a of second filter 11 is closed by closing solenoid valve 20a. In addition, the solenoid valve 17 is set to connect inflow branch 8 directly to substitution-fluid line 10, so that the air pumped by pump 15 is fed into second chamber 9b of first sterile filter 9 and into first chamber 11a of second sterile filter 11 to expel the liquid from the filters. In the event of damage to either one of membranes 9c, 11c separating chambers 9a and 9b and chambers 11a and 11b respectively, air flows along drain line 19 or test line 21, and is detected by sensor 24. More specifically, comparison of the information from sensor 24 with a reference threshold determines the integrity or not of membranes 9c and 11c and, hence, of filters 9 and 11.

Moreover, by acting on solenoid valve 19a, the integrity first of membrane 11c and then of membrane 9c can be tested separately.

Number 30 in Figure 2 indicates as a whole a second dialysis machine (shown only partly).

Parts identical to those of machine 1 are indicated using the same reference numbers, with no further description.

Machine 30 differs from machine 1 by comprising one three-chamber filter 31 as opposed to two sterile filters 9 and 11, which means integrity testing of machine 30 applies to filter 31 and, more specifically, to the two membranes 32 and 33 dividing filter 31 into three chambers 31a, 31b, 31c.

Machine 30 comprises a dialysis solution circuit 34 which differs from that of machine 1 by comprising an inflow branch 35 connected selectively to first chamber 31a or second chamber 31b of filter 31; and a substitution-fluid line 36 connecting third chamber 31c of filter 31 to drip chamber 5.

Inflow branch 35 comprises a bypass solenoid valve 37 which, in test mode, bypasses first chamber 31a of filter 31 to connect inflow branch 35 directly, along a connecting line 39, to second chamber 31b. In normal operating mode, solenoid valve 37 connects inflow branch 35 to first chamber 31a of filter 31, and connecting line 39 connects second chamber 31b of filter 31 to a drain line 38 via a solenoid spill valve 38a.

Machine 30 also comprises a drain line 40 connecting first chamber 31a directly to outflow branch 13, and which is fitted with a solenoid spill valve 40a.

In actual use, once dialysis treatment is completed, machine 30 is switched from dialysis mode to wash/test mode. After dialysis solution circuit 34 has been flushed with an aqueous solution, e.g. the dialysis solution itself, solenoid valve 23 is switched to antibacterial filter 22 to feed circuit 34 with air from antibacterial filter 22 as opposed to the dialysis solution from preparation device 7.

At the same time, the outlet of second chamber 31b of filter 31 is closed by closing solenoid valve 18a and switching bypass solenoid valve 18 to the circuit portion upstream from solenoid valve 18a, and solenoid valve 37 is set to connect inflow branch 35 directly to second chamber 31b of filter 31, so that the air pumped by pump 15 is fed into second chamber 31b of filter 31 to expel the liquid from the filter. In the event of damage to either one of membranes 32, 33, air flows along drain line 40 or test line 21, and is detected by sensor 24. As in machine 1, comparison of the information from sensor 24 with a reference threshold determines the integrity or not of membranes 32 and 33 and, hence, of filter 31.

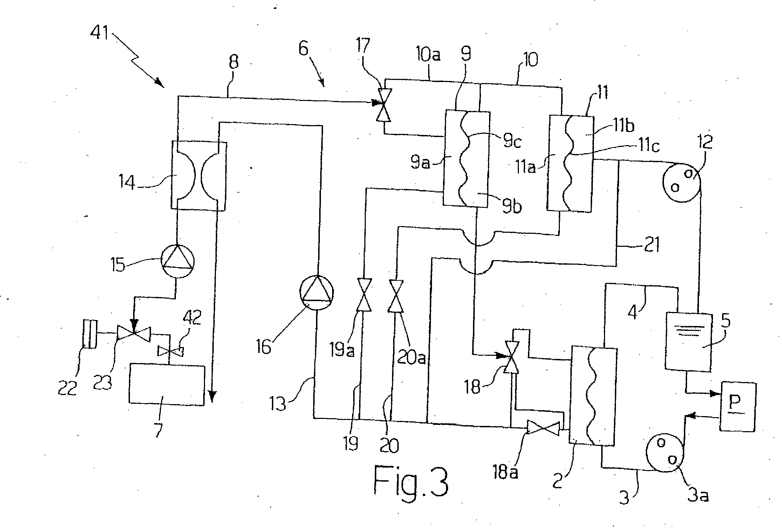

Number 41 in Figure 3 indicates as a whole a third preferred embodiment of the dialysis machine (shown only partly) in accordance with the present invention.

Parts identical to those of machine 1 are indicated using the same reference numbers, with no further description.

Machine 41 differs from machine 1 by having no sensor 24, and by comprising a solenoid valve 42 located between preparation device 7 and solenoid valve 23 to completely cut off inflow branch 8 when solenoid valve 23 is switched to preparation device 7.

In actual use, once dialysis treatment is completed, machine 41 is switched from dialysis mode to wash/test mode. After dialysis solution circuit 6 has been flushed with an aqueous solution, e.g. the dialysis solution itself, solenoid valve 23 is switched to antibacterial filter 22 to feed air into respective chambers 9b and 11a of filters 9 and 11, in the same way as described for machine 1. Once second chamber 9b of filter 9 and first chamber 11a of filter 11 are filled with air, solenoid valve 23 is switched to preparation device 7, and branch 8 is fed with a sufficient amount of a liquid to further compress the air inside chambers 9b and 11a.

At this point, solenoid valve 42 is closed, and flow along branch 8 is measured by differential flow gauge 14. In other words, any damage to either one of membranes 9c, 11c would result in air flow and, consequently, flow of the liquid compressing the air, thus giving a flow reading of other than zero along branch 8.

As will be obvious to anyone skilled in the art, the test method and relative unit according to the present invention are controlled by a known central control unit not described or illustrated.

As will be clear from the above description, the present invention provides for fast, efficient assessment of the integrity of the sterile filters, without recourse to complex, high-cost equipment.

## Claims

1. A method of testing the integrity of a membrane (9c, 11c) of at least one filter (9, 11) located along a dialysis solution circuit (6); said method comprising, in succession, the steps of:
- wetting the test membrane (9c, 11c) with an aqueous solution;
- expelling the liquid from a fill chamber (9b, 11a) bounded by said membrane (9c, 11c); and
- filling said fill chamber (9b, 11a) with a given quantity of gas through an inflow branch (8), after first closing the gas flow lines from said fill chamber (9b, 11a); said method of testing being **characterizing by** comprising the steps of :
- compressing the gas in the fill chamber (9b, 11a) by means of a given amount of a liquid coming through said inflow branch (8) and cutting off completely the inflow branch (8) of said gas and said liquid; and
- detecting the flow of said liquid.

2. A method as claimed in Claim 1, **characterized in that** said detecting operation employs a differential flow gauge (14).

3. A unit for testing the integrity of at least one filter (9, 11) in a dialysis solution circuit (6), comprising
- gas feed means (22, 23, 15) for feeding a gas into a fill chamber (9b, 11a) bounded by a membrane (9c, 11c) of the filter (9, 11) ;
- liquid feed means (7, 15) for feeding a liquid into said fill chamber (9b, 11a);
- a feed line (8) connected to said fill chamber (9b, 11a) acted to carry alternatively both gas and liquid;
- means (18, 18a, 20a) for cutting off gas flow from said fill chamber (9b, 11a);
- a valve (23) for switching the fluid source of feed line (8) from liquid feed means (7) to gas feed means (22) and vice versa;
- means (42) located between feed means (7) and the valve (23) to completely cut off said fed line (8) when the valve (23) is switched to liquid feed means (7); and
- a drain line (13, 19, 21) connected to a chamber (9a, 11b) separated from said fill chamber (9b, 11a) by said membrane (9c, 11c) ;
said unit for testing being **characterized by** comprising a detecting flow device (14) arranged along said feed line (8) and said drain line (13) and able to detect flow of said liquid.

4. A unit as claimed in Claim 3, **characterized in that** said detecting flow device comprise a differential flow gauge (14).

5. A dialysis machine, **characterized by** comprising a filter integrity testing unit as claimed in any one of Claims 3 or 4.

## Patentansprüche

1. Verfahren zum Testen der Intaktheit einer Membran (9c,11c) zumindest eines entlang eines Dialyseflüssigkeitskreislaufs (6) befindlichen Filters (9,11), wobei das Verfahren die aufeinanderfolgenden Schritte umfasst:
- Befeuchten der Testmembran (9c,11 c) mit einer wässrigen Lösung;
- Ausstoßen der Flüssigkeit aus einer von der Membran (9c, 11c) begrenzten Füllkammer (9b,11a); und
- Füllen der Füllkammer (9b,11a) mit einer gegebenen Menge an Gas durch eine Einflussleitung (8) nach vorherigem Schließen der Gasflussleitungen aus der Füllkammer (9b, 11a);
wobei das Testverfahren **dadurch gekennzeichnet ist, dass** es die folgenden Schritte umfasst:
- Komprimieren des Gases in der Füllkammer (9b,11a) durch Zufluss einer gegebenen Menge an Flüssigkeit durch die Einflussleitung (8) und komplettes Schließen der Einflussleitung (8) des Gases und der Flüssigkeit; und
- Erfassen des Flusses der Flüssigkeit.

2. Verfahren nach Anspruch 1, **dadurch gekennzeichnet, dass** bei dem Erfassungsvorgang ein Durchflussdifferenzmessgerät (14) verwendet wird.

3. Einheit zum Testen der Intaktheit zumindest eines Filters (9,11) in einem Dialyseflüssigkeitskreislauf (6) mit
- Gaseinspeisungsmitteln (22,23,15) zum Einspeisen von Gas in eine Füllkammer (9b,11a), welche durch eine Membran (9c,11c) des Filters (9,11) begrenzt ist;
- Flüssigkeitseinspeisungsmitteln (7,15) zum Einspeisen von Flüssigkeit in die Füllkammer (9b,11a);
- einer mit der Füllkammer (9b,11a) verbundenen Einspeisungsleitung (8) zum alternativen Führen von sowohl Gas als auch Flüssigkeit;
- Mitteln (18,18a,20a) zum Abstellen des Gasflusses aus der Füllkammer (9b,11a);
- einem Ventil (23) zum Umschalten der Fluidquelle der Einspeisungsleitung (8) von Flüssigkeitseinspeisungsmitteln (7) auf Gaseinspeisungsmittel (22) und umgekehrt;
- zwischen den Einspeisungsmitteln (7) und dem Ventil (23) befindliche Mittel (42) zum kompletten Schließen der Einspeisungsleitung (8), wenn das Ventil (23) auf Flüssigkeitseinspeisungsmittel (7) umgeschaltet ist; und
- einer Abflussleitung (13,19,21), welche mit einer von der Füllkammer (9b,11a) durch die Membran (9c,11c) abgetrennten Kammer (9a,11b) verbunden ist;
wobei die Testeinheit **dadurch gekennzeichnet** ist, das sie eine Durchflusserfassungsvorrichtung (14), welche entlang der Einspeisungsleitung (8) und der Abflussleitung (13) angeordnet ist und den Fluss der Flüssigkeit messen kann, umfasst.

4. Einheit nach Anspruch 3, **dadurch gekennzeichnet, dass** die Durchflusserfassungsvorrichtung ein Durchflussdifferenzmessgerät (14) umfasst.

5. Dialysegerät, **dadurch gekennzeichnet, dass** es eine Einheit zum Testen der Intaktheit eines Filters nach einem der Ansprüche 3 oder 4 umfasst.

## Revendications

1. Procédé de test de l'intégrité d'une membrane (9c, 11c) d'au moins un filtre (9, 11) situé le long d'un circuit de solution de dialyse (6) ; ledit procédé comprenant, successivement, les étapes qui consistent :
- à mouiller la membrane de test (9c, 11c) avec une solution aqueuse ;
- à expulser le liquide à partir d'une chambre de remplissage (9b, 11a) délimitée par ladite membrane (9c, 11c) ; et
- à remplir ladite chambre de remplissage (9b, 11a) avec une quantité donnée de gaz à travers un branchement d'admission (8), après une première fermeture des conduites d'écoulement de gaz à partir de ladite chambre de remplissage (9b, 11a) ; ledit procédé de test étant **caractérisé en ce qu'**il comprend les étapes qui consistent :
- à comprimer le gaz dans la chambre de remplissage (9b, 11a) au moyen d'une quantité donnée d'un liquide circulant à travers ledit branchement d'admission (8) et à fermer complètement le branchement d'admission (8) dudit gaz et dudit liquide ; et
- à détecter l'écoulement dudit liquide.

2. Procédé tel que revendiqué dans la revendication 1, **caractérisé en ce que** ladite opération de détection utilise un manomètre différentiel (14).

3. Unité de test de l'intégrité d'au moins un filtre (9, 11) dans un circuit de solution de dialyse (6), comprenant
- un moyen d'alimentation en gaz (22, 23, 15) destiné à alimenter un gaz dans une chambre de remplissage (9b, 11a) délimitée par une membrane (9c, 11c) du filtre (9, 11) ;
- un moyen d'alimentation en liquide (7, 15) destiné à alimenter un liquide dans ladite chambre de remplissage (9b, 11a) ;
- une conduite d'alimentation (8) reliée à ladite chambre de remplissage (9b, 11a) conçue pour transporter, en alternance, à la fois le gaz et le liquide ;
- un moyen (18, 18a, 20a) destiné à couper l'écoulement du gaz à partir de ladite chambre de remplissage (9b, 11a) ;
- une soupape (23) destinée à faire basculer la source de fluide de la conduite d'alimentation (8) du moyen d'alimentation en liquide (7) vers le moyen d'alimentation en gaz (22) et inversement ;
- un moyen (42) situé entre le moyen d'alimentation (7) et la soupape (23) pour fermer complètement ladite conduite d'alimentation (8) lors du basculement de la soupape (23) vers le moyen d'alimentation en liquide (7) ; et
- une conduite d'évacuation (13, 19, 21) reliée à une chambre (9a, 11b) séparée de ladite chambre de remplissage (9b, 11a) par ladite membrane (9c, 11c) ;
ladite unité de test étant **caractérisée en ce qu'**elle comprend un dispositif de détection d'écoulement (14) agencé le long de ladite conduite d'alimentation (8) et de ladite conduite d'évacuation (13) et apte à détecter l'écoulement dudit liquide.

4. Unité telle que revendiquée dans la revendication 3, **caractérisée en ce que** ledit dispositif de détection d'écoulement comprend un manomètre différentiel (14).

5. Machine de dialyse, **caractérisée en ce qu'**elle comprend une unité de test de l'intégrité d'un filtre telle que revendiquée dans l'une quelconque des revendications 3 ou 4.
